# EUROPEAN PATENT APPLICATION

(11) **EP 4 647 050 A1**
(43) Date of publication of application: **12.11.2025**
(21) Application number: 24174550.4
(22) Date of filing: 07.05.2024
(51) Int. Cl.: A61F 5/451, A61M 25/00

(54) **URINARY CATHETER KIT WITH URINE COLLECTION BAG**

(71) Applicant: Wellspect AB, 431 21 Mölndal (SE)
(72) Inventor: LOVMAR, Martin, 431 69 Mölndal (SE); MOLINDER, Hanna, 411 27 Göteborg (SE); ERKÉN, Simon, 903 31 Umeå (SE)
(74) Representative: Aldridge, Henry Alexander

(57) **Abstract**

The present disclosure relates to a urinary catheter kit and a method for manufacturing the urinary catheter kit. The urinary catheter kit comprises a urinary catheter with a catheter shaft extending along an axial direction, at least one eyelet arranged at a proximal insertion end, and a handle arranged at an opposite distal end of the catheter shaft, wherein said handle defines a cavity. The catheter kit further comprises a urine collection bag made of a flexible material. The urine collection bag has a collection bag opening which is attached to the handle so as to enable the urine collection bag to communicate with a central lumen of the catheter shaft via said handle. Wherein the urine collection bag, in a storage state, is compacted and at least partially housed inside the cavity of the handle.

## Description

### TECHNICAL FIELD OF THE INVENTION

The present invention relates to a urinary catheter kit comprising a urinary catheter and a urine collection bag.

### BACKGROUND OF THE INVENTION

Urinary catheters are widely used for intermittent catheterization. In intermittent catheterization, the urinary catheter is inserted into the bladder (e.g. via the urethra or a stoma) and urine is discharged via the catheter and the catheter is then removed. Urinary catheters typically comprise a hollow catheter shaft which is adapted for insertion through the urethra, and a connector, which is to remain outside the urethra, and allow the urine to exit from the hollow catheter shaft.

To this end, intermittent urinary catheters can be used to void urine directly from the bladder into e.g. a toilet, into a separately provided urine collection bag or into a urine collection bag which is connected or connectable to the urinary catheter and provided as a compact "kit" together with the urinary catheter.

Since a toilet or a drain is not always available, or it may be challenging for some users to orient themselves so as to discharge urine directly into a toilet or drain, many users prefer to use intermittent urinary catheters provided as a kit with a urine collection bag over other types of urinary catheters. To enable urine to drain from the bladder into the urine collection bag when the user performs catheterization the urine collection bag of a kit may be configured to be manually held or connected to the catheter connector or, alternatively, the urine collection bag is prearranged on the connector. After the bladder has been drained into the urine collection bag, the user can dispose of both the urine collection bag and the urinary catheter. In some cases, the urine collection bag is arranged to be torn opened so as to enable the urine collection bag to be emptied (e.g. into a toilet) prior to disposing the emptied urine collection bag.

Various catheter kit solutions have been proposed where the urine collection bag is packaged together with the urinary catheter in different ways, with the aim of providing a kit product which is small, light and easy to use. For example, to make the kit small and light, some solutions involve an external package which acts as both an external barrier which protects the urinary catheter and as a urine collection bag during use.

However, despite recent attempts to provide compact kits comprising a urinary catheter and a urine collection bag, the current kits are still bulkier than desired and may be difficult for some users to use properly. For example, while an external package which doubles as a urine collection bag may result in a fairly compact kit, the fact that the external package must be configured to both offer sufficient protection for the urinary catheter (which e.g. may require a thicker and more resilient plastic material) and also be dimensioned to house a sufficient amount of urine may make the external package bulky. Since the urine collection bags need to be able to hold a certain amount of urine (e.g. at least the amount of urine typically voided from the bladder) the urine collection bags are often large, and in solutions where the urine collection bag is to be provided inside a package alongside the urinary catheter, it is often required to fold the urine collection bag multiple times and provide extra retention elements (such as clips or rubber bands) to retain the bag in a compact storage configuration when it is inside the package. The additional retention elements further add to the total weight and size of the kits and provision of additional retention elements may make the catheter kit more expensive and more complicated to manufacture, which may lead to a more expensive final product. Additionally, the necessary removal of one or more retention elements prior to use, to allow the urine collection bag to be unfolded, may be difficult for some users.

There is also a general need for smaller and more compact catheter kit products, which are easier to carry around, easier to handle and use in a discrete manner, easier to discretely discard after use, etc.

To this end, there is a need for an improved kit comprising a urine collection bag and a urinary catheter which overcomes at least some of the shortcomings mentioned in the above.

### SUMMARY OF THE INVENTION

It is the purpose of the present disclosure to present an improved urinary catheter kit, comprising a urinary catheter and a urine collection bag, wherein the urinary catheter kit is both easy to use and more compact, compared to previous solutions.

According to a first aspect of the present invention there is provided a urinary catheter kit comprising a urinary catheter. The urinary catheter comprises a catheter shaft extending along an axial direction, from a proximal insertion end to a distal end, wherein said catheter shaft comprises a central lumen, at least one eyelet arranged at the insertion end, wherein said eyelet communicates with the central lumen, and a handle arranged at the distal end of the catheter shaft, wherein said handle defines a cavity having an inner cavity opening which is in fluid communication with the central lumen. The urinary catheter kit further comprises a urine collection bag made of a flexible material, wherein the urine collection bag has a collection bag opening which is attached to the handle so as to enable the urine collection bag to communicate with the central lumen via the inner cavity opening and wherein the urine collection bag, in a storage state, is compacted and at least partially housed inside the cavity of the handle.

In the storage state, the urine collection bag is folded, rolled or otherwise compacted so as to at least partially fit inside the cavity of the handle. The urine collection bag can be withdrawn from the handle and extended to an extended state prior to catheterization. In the extended state, the urine collection bag is at least partially unfolded, unrolled or otherwise extended so as to be in a state in which it is ready to be filled with urine.

With a handle which is configured to at least partially house the urine collection bag in a compacted state, a compact and easy to use catheter kit is provided without any loose parts and without any need for assembling and disassembling the parts. The handle of the urinary catheter of the first aspect of the invention may be larger compared to traditional handles for urinary catheters. The handles are also sometimes referred to as "connectors" since they are configured to be connected to an extension tube or a urine collection bag.

Advantageously, the urine collection bag is not fully housed inside the cavity but protrudes outside of the cavity of the handle to make it visible and easier to grip and pull out, which makes a product which is generally easier and more convenient to use.

Surprisingly, the catheter handle of the first aspect may however still be sufficiently small so as to fit inside conventional catheter packages while still being able to house the urine collection bag. Additionally, since a handle (or connector) is typically present on urinary catheters, there is no need for additional retention elements (such as rubber bands or clips) for holding the urine collection bag in a compacted state, which makes the catheter kit easier to use, makes the catheter kit require less material to manufacture and produces less waste during use. The catheter kit is also compact, making it easier for users to bring the catheter kit along.

Furthermore, besides the fact that the handle is slightly larger compared to the handle (or connector) of traditional urinary catheters, the urinary catheter kit will generally look and feel similar to traditional catheters which is advantageous for many users. Additionally, a slightly larger handle may be advantageous since a larger handle may in general be easier to grip. A larger handle may therefore be especially beneficial for users with decreased finger dexterity.

The urine collection bag is hereby also protected by the handle prior to use, when in the compacted state, thereby alleviating the risk of inadvertent damaging of the bag.

The handle preferably has a cross-sectional dimension in a plane perpendicular to the axial direction which exceeds the cross-sectional dimensions of the catheter shaft.

The purpose of the handle is to be sufficiently large to encompass a cavity configured to at least partially house the urine collection bag and to provide a reliable grip for the user, so that the user can hold the catheter by the handle even if e.g. the handle is wet or the user's fingers are wet. It is understood that there are many possible designs of the handle which achieves these purposes. For example, the handle may have a generally rounded cross-sectional shape which is comfortable to grip. As another example, the outer surface of the handle may be provided with an outer surface with indentations and/or protrusions that form a textured surface that is easy to grip.

The handle may be produced as a separate component and be attached to the catheter shaft by welding, gluing or the like. However, the handle may also be integrally formed with the catheter shaft. In an embodiment, the handle and catheter shaft are integrally formed (monolithically integrated).

In some implementations the urinary catheter may be a hydrophilic catheter. That is, at least the catheter shaft may be coated with a hydrophilic coating which becomes slippery when wetted with a wetting fluid. When wetted with a wetting fluid, hydrophilic catheters may be more comfortable to use compared to other types of urinary catheters.

In some implementations, the urine collection bag opening is attached to an inner surface of the cavity of the handle.

Accordingly, in the compacted storage state a portion of the urine collection bag, as well as its attachment to the handle, is hidden inside the cavity of the handle. A large portion of the external surface of the handle (e.g. the entire outer surface of the handle) therefore remains exposed, allowing the user to grip the outer surface of the handle directly.

Alternatively to attach the urine collection bag to the inner surface of the cavity of the handle, the urine collection bag opening may be attached to an outer surface of the handle.

Attaching the urine collection bag to the outer surface may facilitate fitting more of the urine collection bag inside the cavity. Additionally, attachment of the urine collection bag to the outer surface may be easier to realize during manufacturing.

In some implementations, the handle and the cavity extend in the axial direction and the cavity has a first cross-sectional dimension in a first direction perpendicular to the axial direction and second cross-sectional dimension in a second direction perpendicular to the axial direction and the second direction, wherein the first cross-sectional dimension is larger than the second cross-sectional dimension. In general, the inner diameter of the cavity may vary and may not be constant which would be the case if the cavity is circular.

For example, the cross-sectional shape of the cavity is not rotationally symmetric and may be oval, elliptical, or lens shaped (i.e. the shape of the intersection between two offset circles of equal or unequal radii).

The cross-sectional shape of the outer surface may be the same as that of the cavity. Alternatively, the cross-sectional shape of the outer surface may be different from that of the cavity. It has been found that a handle and/or cavity which has an oval cross-section remains easy to grip while allowing a larger urine collection bag to fit inside the cavity.

For example, when the outer surface has a diameter which varies a user may grip the handle at the smallest diameter as this may facilitate reliably gripping the handle so as to accidentally drop the catheter kit.

In some implementations, the urinary catheter kit further comprises a backflow prohibiting element configured to prevent or limit a liquid flow from the urine collection bag into the central lumen of the catheter shaft, while allowing a liquid flow from the central lumen of the catheter shaft into the urine collection bag.

To prohibit or prevent urine from flowing backwards (i.e. from the urine collection bag into the central lumen of the catheter shaft) a backflow prohibiting element may be placed in the catheter shaft, in the handle or in the urine collection bag.

For example, the backflow prohibiting element may comprise or consist of a unidirectional valve (also known as a one-way valve) which only allows liquids to flow in one direction through the valve. The unidirectional valve may be arranged in the catheter shaft or the handle so as to prohibit or prevent urine from flowing backwards, from the urine collection bag into the central lumen of the catheter shaft.

In some implementations, the backflow prohibiting element comprises or consists of a flexible inner sleeve circumferentially attached to the urine collection bag at a proximal end, wherein, when the urine collection bag is in an extended state, a distal end of the flexible inner sleeve, which is opposite to said proximal end, is free inside the urine collection bag.

That is, one example of a simple yet effective backflow prohibiting element is a flexible inner sleeve arranged inside the urine collection bag. The flexible inner sleeve is arranged such that fluid entering through the handle into the urine collection bag must flow through the flexible inner sleeve, in a direction going from the proximal end towards the distal end. In this way, liquids may easily flow through the flexible inner sleeve in one direction, but it is comparatively difficult for liquid to flow in the opposite direction since the liquid will tend to push the distal end of the flexible inner sleeve to the side, which traps the liquid.

In some implementations, the urine collection bag comprises a neck section and the flexible inner sleeve is arranged at least partially inside said neck section.

A neck section may also be used without the flexible inner sleeve. A neck section is a comparatively narrow section of the urine collection bag which is located between the handle of the catheter and a main storage portion of the urine collection bag. A neck section may make it easier to compact the urine collection bag in a way such that it fits inside the cavity of the handle and at the same time may make the catheter kit easier to use since it allows the main storage portion to remain at some distance from the urinary catheter during use.

Provision of the flexible inner sleeve in the neck portion means that the urine inside the main storage portion is prohibited or prevented from flowing backwards, into the handle or the central lumen of the catheter shaft.

In some implementations, the flexible inner sleeve is integrally formed with the urine collection bag.

The flexible inner sleeve may be made from the same material as the urine collection bag, which allows for efficient manufacturing. Additionally, since the flexible inner sleeve is made of a flexible material, it can easily be compacted together with the urine collection bag so as to be stored inside the cavity of the handle. In one example implementation, the urine collection bag is manufactured with an extension (at a proximal part of the neck portion) wherein the extension is folded inwards to form the flexible inner sleeve as an integral part of the urine collection bag.

In some implementations, the flexible inner sleeve has an inner diameter which decreases towards the distal end.

Accordingly, the flexible inner sleeve may be generally shaped like a funnel, with the narrow opening arranged in at the distal end. With this type of flexible inner sleeve, the backwards flow prevention function is improved.

In some implementations, the handle extends in the axial direction, and the largest axial length of the cavity is at least 2.5 cm, at least 3 cm or at least 3.5 cm and/or the largest cross-sectional dimension of the cavity, in a plane perpendicular to the axial direction, is at least 1 cm, or at least 1.5 cm.

These handle dimensions allow the urine collection bag to be stored inside the handle, while still making the catheter kit compact and possible to house inside already existing external packages.

In some implementations, the urine collection bag, when in an extended state, comprises a neck portion and a main storage portion, wherein a transverse width of the urine collection bag in the main storage portion is greater than a transverse width of the neck portion and wherein the neck portion is arranged proximally of the main storage portion, between the collection bag opening and the main storage portion.

A urine collection bag with a comparatively narrow neck portion and a comparatively wide main storage portion may make the urine collection bag easier to compact so as to fit inside the handle cavity. Additionally, by connecting the main storage portion to the handle via the neck portion the catheter kit may be more convenient to use. For example, without the neck portion the comparatively wide main storage portion is located in direct handle with the handle, whereby the urine collection bag may be in the way during use.

In some implementations, the main storage portion has, to reach the storage state, been folded at least once along a transverse folding line, the transverse folding line being perpendicular to the axial direction when the urine collection bag is in the extended state and subsequently rolled, or repeatedly folded, along a roll axis parallel to the axial direction.

This type of compactization has been shown to result in a very compact urine collection bag which can easily fit inside the cavity of the handle.

In some implementations, the urine collection bag further comprises at least one tear line configured to enable the urine collection bag to be torn open.

The at least one tear line may be used to form an opening, allowing the urine collection bag to be emptied. The at least one tear line may be provided at a variety of locations and it is envisaged that more than one tear line is provided, giving the user the choice of where to tear the bag open. In one implementation, at least one tear line is provided in (e.g. a proximal end of) the neck portion of the urine collection bag, allowing the main storage portion to be separated from the handle, whereby the urine collection bag can be emptied through the neck portion and/or the neck portion can be tied to seal the urine inside the urine collection bag. Additionally or alternatively, at least one tear line is provided at the main storage portion allowing the main storage portion to be opened and urine drained therefrom (optionally while the neck portion is still attached to the handle, allowing the kit to be held by the handle).

According to a second aspect of the invention, there is provided a urinary catheter assembly, comprising an external package and a catheter kit according to the first aspect, arranged inside said external package.

The external package is configured to enclose the catheter kit and e.g. keep it sterile. This allows a user to conveniently bring the package with him or her and when needed open the external package and remove the catheter kit from the external package. When located inside the external package, the urine collection bag is in its compacted storage stage and housed at least partially inside the handle of the catheter. Hereby, the catheter package can be made very compact, not much larger than traditional urine catheter packages used for urinary catheters without a urine collection bag, while also comprising a urine collection bag.

According to a third aspect of the invention, there is provided a method for manufacturing a handle for a catheter kit. The method comprises providing a handle configured to be arranged at the distal end of a catheter shaft, wherein said handle defines a cavity having an inner cavity opening which is configured to be in fluid communication with a central lumen of the catheter shaft, providing a urine collection bag comprising a collection bag opening, attaching the collection bag opening to the handle of the urinary catheter, compacting the urine collection bag and arranging the compacted urine collection bag inside the cavity of the handle.

The step of compacting the urine collection bag may comprise at least one of folding the urine collection bag and rolling the urine collection bag.

The invention according to the second and third aspect features the same or equivalent benefits as the invention according to the first aspect.

### BRIEF DESCRIPTION OF THE DRAWINGS

The present invention will be described in more detail with reference to the appended drawings, showing currently preferred embodiments of the invention.
Fig. 1 shows schematically a catheter kit with a compacted urine collection bag according to some implementations.
Fig. 2a-d show a perspective view, a cross-sectional view and a side view of several types of handles according to some implementations.
Fig. 3 shows schematically a handle according to some implementations compared to a traditional handle.
Fig. 4 shows a catheter kit with a urine collection bag in its extended state, according to some implementations.
Fig. 5a and 5b illustrate a cross-section of a handle attached to the urine collection bag according to some implementations.
Figs. 6a-i illustrate how a flexible inner element acting as a backflow prohibiting element can be formed and how the flexible inner element can prevent or limit backwards flow, according to some implementations.
Figs. 7a-c are cross-sections of a handle and a urine collection bag with an integrated flexible inner sleeve according to some implementations.
Figs. 8a-f illustrate how the urine collection bag can be compacted according to some implementations.
Figs. 9a-e illustrate a cross-section of differently shaped urine collection bags according to some implementations.
Fig. 10 is a flow chart illustrating a method for manufacturing a catheter kit according to some implementations.

### DETAILED DESCRIPTION OF CURRENTLY PREFERRED EMBODIMENTS

In the following detailed description, preferred embodiments of the invention will be described. However, it is to be understood that features of the different embodiments are exchangeable between the embodiments and may be combined in different ways, unless anything else is specifically indicated. It may also be noted, for the sake of clarity, that the dimensions of certain components illustrated in the drawings may differ from the corresponding dimensions in real-life implementations of the invention.

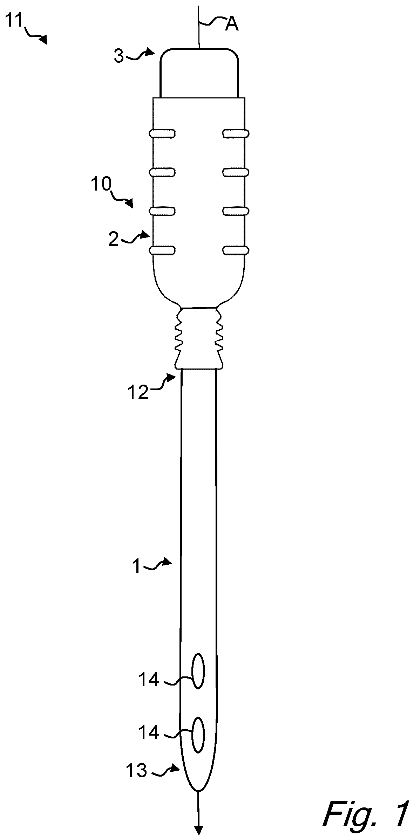

Fig. 1 illustrates a catheter kit 11 according to some implementations of the present invention. The catheter kit 11 comprises a urinary catheter 10. The urinary catheter 10 comprises an elongated catheter shaft 1 extending along an axial direction A. The catheter shaft 1 extends from a proximal insertion end 13 to an opposite distal end 12.

The terms "proximal" and "distal" are in this disclosure used to describe the proximity of parts of the catheter to the user when he or she uses the catheter for self-catheterization. The proximal end 13 of the catheter shaft 1 is inserted first through the urethra of the user whereby this part of the urinary catheter 10 is closer to, i.e. more proximal to, the bladder of the user during self-catheterization.

The proximal insertion end 13 of the catheter shaft 1 may comprise a rounded tip to make insertion of the catheter shaft 1 into the urethra more comfortable.

The catheter shaft 1 is hollow and comprises a central lumen configured to allow urine from the bladder of a patient to flow through the catheter shaft, through the central lumen. To allow urine to enter the central lumen the catheter comprises one or more apertures (often referred to as eyelets 14 that communicate with the central lumen, allowing urine to enter into the central lumen of the catheter shaft.

The catheter shaft may be made of a plastic material. For example, the catheter shaft 1 is made of a thermoplastic material, e.g. a thermoplastic polymer or thermoplastic elastomer.

A hydrophilic coating may be arranged on the catheter shaft 1, at least over an insertable length thereof. Alternatively, the catheter shaft 1 may be formed by a hydrophilic material. The hydrophilic coating or material provides a very low friction to the catheter shaft 1 when wetted.

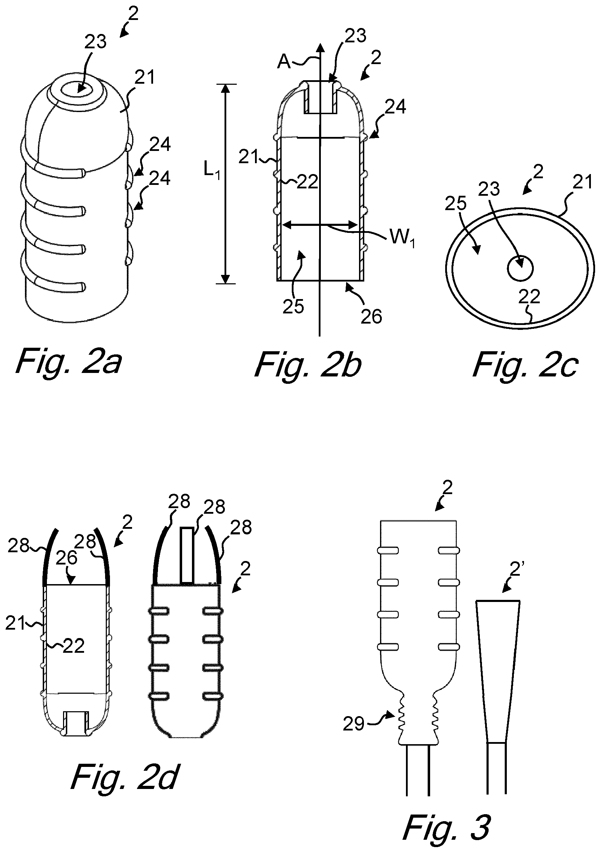

The distal end 12 of the catheter shaft 1 is connected to a handle 2. With further reference to fig. 2a-c the handle 2 according to some implementations is shown in more detail. The handle 2 has a cavity 25 and the handle 2 is further provided with an inner cavity opening 23 allowing the central lumen of the catheter shaft 1 to fluidly communicate with the cavity 25. The inner cavity opening 23 may e.g. be sized and adapted to receive the entire catheter shaft 1 whereby the central lumen of the catheter shaft 1 gets into fluid communication with the cavity 25. The cavity 25 further comprises an outer cavity opening 26.

Fig. 3 shows another example of the handle 2 according to some implementations. The handle 2 of fig. 3 is generally similar to that of fig. 2a-c but the handle of fig. 3 further comprises an axial extension 29 wherein the catheter shaft is attached to the extension 29. The exemplary handle designs shown in fig. 2a-c and fig. 3 are only two out of many possible handle designs that are easy to grip as well as sized and adapted to house the urine collection bag in the compacted state.

The handle 2 may be integrally formed with the catheter shaft 1 or manufactured separately and attached to the distal end 12 of the catheter shaft 1. The handle 2 may also be made of a plastic material. For example, the handle 2 is made of a polymer material. The handle 2 may be rigid (e.g. made of hard plastic material) but it is also envisaged that the handle is soft and elastically deformable, whereby the handle 2 may be made of a rubber material. It also envisaged that the handle 2 is made of a biobased material such as a paper material or another cellulose-based material.

The handle 2 has an outer surface 21 and an opposite inner surface 22 delimiting the cavity 25. In fig. 2a a perspective view of the handle 2 according to some implementations is shown, the outer surface 21 of the handle 2 may be rounded and without sharp edges, such that the handle 2 is comfortable to grip. The outer surface 21 of the handle 2 is preferably configured to make it easy to grip and held between two fingers. To this end, the handle 2 may be provided with a textured outer surface 21 which makes the handle 2 easier to grip. For example, the outer surface 21 of the handle 2 is provided with one or more protrusions 24 which extend at least partially around the circumference of the handle 2 so to form one or more ribs, as shown in fig. 2a. It is however understood that this type of protrusion 24 is only one example of surface features which may be added to the outer surface to make it a textured surface. As further examples, the outer surface is provided with indentations, an undulating or corrugated surface profile and/or other types of protrusions (such as small semi-spheres) forming a textured surface that is easy to grip.

The outer surface 21 of the exemplary handle 2 of figs. 2a-c tapers inwards towards proximal end of the handle 2, i.e. where the inner cavity opening 23 is provided. Accordingly, the handle 2 of these examples extends from the comparatively large outer cavity opening 26 to the comparatively smaller inner cavity opening 23. Additionally or alternatively, to facilitate better retention of the urine collection bag it is also envisaged that the handle 2 may taper towards a smaller diameter at the outer cavity opening 26 from a larger central opening to e.g. form a lip or rim that clasps the urine collection bag when it is housed therein.

Fig. 2b shows a cross-sectional view of the handle 2. Opposite the outer surface 21 the handle 2 has an inner surface 22 which at least partially delimits the cavity 25. The cavity 25 is in fluid communication with a central lumen of the catheter shaft via the inner cavity opening 23. The cavity 25 further comprises an outer cavity opening 26 opposite the inner cavity opening 23 making the handle 2 generally tube-shaped, extending from the inner cavity opening 23 to the outer cavity opening 26. The outer cavity opening 26 is in some implementations larger than the inner cavity opening 23. For example, a cross-sectional dimension of the outer cavity opening 26 is at least 120%, at least 150% or at least 200% of a cross-sectional dimension of the inner cavity opening 23. The purpose of the inner cavity opening 23 is to allow urine to enter into the cavity 2 and optionally to retain the catheter shaft 1. To this end, the inner cavity opening 23 can be made comparatively small.

On the other hand, the outer cavity opening 26, and the cavity 25 as such, are made comparatively large so as to be able to at least partially house a compacted urine collection bag, in its storage state, inside the cavity 25.

For example, the handle 2 has a largest length L₁ along the axial direction A which is at least 2 cm, at least 2.5 cm, at least 3 cm or at least 3.5 cm. The cavity 25 may occupy substantially the entire handle 2 whereby the cavity 25 has a length along the axial direction A which is substantially the same as the length L₁ of the handle 2.

The cavity 25 may further have a largest cross-sectional width W₁ in a plane perpendicular to the axial direction A wherein the width W₁ is at least 1 cm, at least 1.5 cm or at least 2 cm.

In some implementations, to provide a sufficiently large volume inside the cavity 25, allowing the cavity 25 to house at least a portion of a urine collection bag inside the cavity 25, the cavity 25 may have a width W₁ of at least 1 cm over a region that extends at least 2 cm along the axial direction A or a width W₁ of at least 1.2 cm over a region that extends at least 3 cm along the axial direction A.

To make the handle 2 comfortable to grip and space efficient it may be realized with a rounded outer profile, having e.g. an oval cross-sectional shape. However, it is of course possible to utilize a handle 2 which has a different shape, such as a polygonal (e.g. triangular, square or octagonal) outer profile. In fig. 2c a perspective view of the handle 2 is shown as seen along the axial direction A, looking into the cavity 25 via the outer cavity opening 26 towards the inner cavity opening 23. The cross-sectional profile of the outer surface 21 and inner surface 22 of the handle 2 may be any shape such as generally rounded, oval or polygonal. In the embodiment shown in fig. 2c the cross-sectional profile of the outer surface 21 and inner surface 22 are oval as this has been found to make it easier to fit the urine collection bag inside the handle 2 while still ensuring that the handle 2 is as small as possible while also being easy to grip.

To provide an as large volume in the cavity 25 as possible in an as small handle 2 as possible, the cross-sectional profile of the outer surface 21 may generally follow the cross-sectional profile of the inner surface 22 allowing the handle 2 to be made with a material having a substantially equal material thickness. However, it is envisaged that by utilizing a material thickness which varies circumferentially around the axial direction A it is possible to realize a handle 2 having a different cross-sectional profile of the outer surface 21 compared to the cross-sectional profile of the inner surface 22. For example, the cross-sectional profile of the outer surface 21 may be oval or circular wherein the cross-sectional profile of the inner surface 22 is polygonal, e.g. octagonal.

Fig. 2d shows a cross-sectional view and a side view of a holder 2 provided with retention arms 28 (or claws) arranged around the outer cavity opening 26 according to some implementations. The retention arms 28 may be elastically flexible and serving to retain the urine collection bag inside the handle 2 while still making the urine collection bag easy to grip (e.g. between neighboring retention arms 28) and pull out.

While the handle 2 is depicted as a solid piece of material it is envisaged that at least a part (and advantageously a distal part) of the handle 2 may be realized as a net or mesh structure comprising one or more openings or slits (in addition to the outer cavity opening 26) extending through the material of the handle 2. With a net or mesh material the handle 2 may become more flexible and easier to grip. As long as a portion of the handle 2 is solid to allow liquid tight attachment of the urine collection bag, a portion of the handle 2 may hereby be realized with multiple openings to make the handle more flexible and reduce the amount of material required to manufacture the handle 2. For example, the handle 2 may be provided with a distal crown portion with one or more openings or slits that help retain the urine collection bag while making at least this portion of the connector more flexible. The retention arms 28 of fig. 2d may be seen as one example of a distal crown portion with a plurality of slits (i.e. the space between the retention arms 28).

Turning to fig. 3 a handle 2 according to aspects of the present invention is compared to a typical handle/connector 2' of intermittent urinary catheters. The handle 2 is generally larger than a typical handle/connector 2' in both width and length to allow the handle 2 to house at least a part of the urine collection bag. A larger handle 2 may be easier to grip, making the catheter kit of the present invention easier to use for most users. At the same time, compared to a catheter kit comprising a traditional catheter with a traditional handle/connector 2' and a separately packed or compacted urine collection bag, the larger handle 2 will make for a more space efficient and compact all-in-one catheter kit since the urine collection bag can at least partially be housed inside the connector.

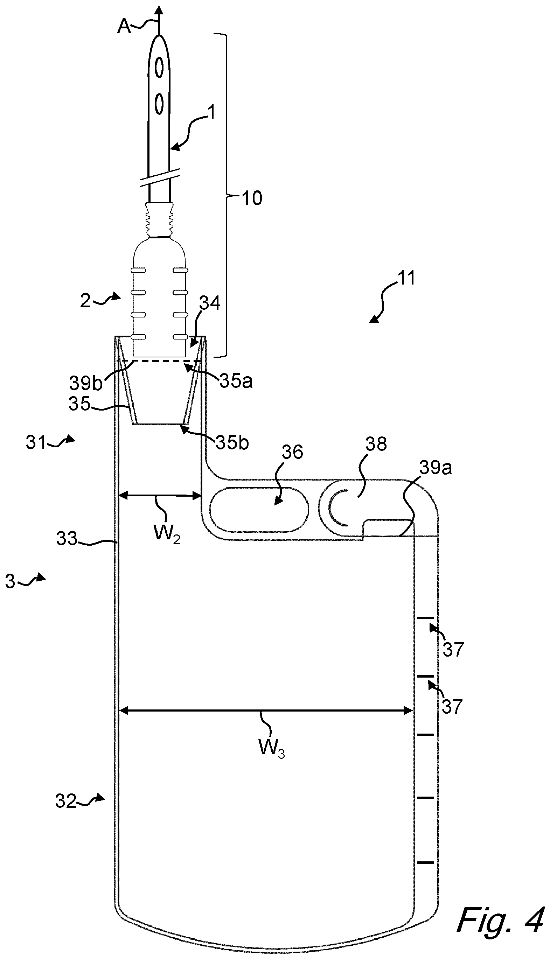

Fig. 4 shows a catheter kit 11 with a urine collection bag 3 in its extended state according to some implementations.

The urine collection bag 3 may be made from a thin flexible material. For example, the urine collection bag 3 is made from a polymer material such as polyethylene or polypropylene. The urine collection bag 3 may be made of a plastic film. For example, the urine collection bag 3 is made by holding two film layers against each other and welding or gluing the film layers together along a path to form the desired shape of the urine collection bag 3. As a further example, the urine collection bag 3 is made by folding a single film layer and welding or glueing the folded film layer along a path to form a urine collection bag 3 of the desired shape. Hereby, the urine collection bag may be formed by one or more film layers which have been folded, welded and/or glued to form the urine collection bag 3.

Accordingly, the urine collection bag 3 may have a material weld 33 going around the circumference of the urine collection bag 3. A portion of the urine collection bag is however devoid of a material weld 33 to form the collection bag opening 34.

The urine collection bag 3 is configured to house a predetermined amount of liquid. For example, the urine collection bag is configured to hold at least 400 ml of liquid, at least 600 ml of liquid or at least 800 ml of liquid. The urine collection bag 3 may be provided with markings 37 allowing a user to identify the amount of urine inside the urine collection bag 3 after catheterization.

Many different shapes of the urine collection bag 3 are envisaged which still enables the urine collection bag 3 to hold a sufficient amount of urine, and to be compacted into a compact storage state and to be stored at least partially inside the handle 2.

The urine collection bag 3 of fig. 4 comprises a comparatively narrow neck portion 31 which comprises the collection bag opening 34 in proximal portion thereof. The collection bag opening 34 is connected to the handle 2 so as to establish fluid communication between the central lumen of the catheter shaft 1 and the urine collection bag 3 via the cavity inside the handle 2. The distal portion of the neck portion 31 communicates with a main storage portion 32 of the urine collection bag 3.

The main storage portion 32 may have a transverse width W₃ which is greater than a transverse width W₂ of the neck portion 31. The transverse widths W₂, W₃ are measured perpendicular to the axial direction A when the urine collection bag 3 is in its extended state.

The comparatively narrow neck portion 31 serves to guide urine into the main storage portion 32 and the provision of a comparatively narrow neck portion 31 further facilitates space efficient compactization of the urine collection bag 3 as will be described below. The neck portion 31 further allows the main storage portion 32 to be at some distance from the handle 2, allowing the main storage portion 32 to e.g. hang from the handle 2 via the neck portion 31 during catheterization which may make the catheter 10 more convenient to use.

In some implementations, the main storage portion 31 has a transverse width W₃ in the extended state which is at least 200%, at least 250%, at least 300% or at least 350% of the transverse width W₂ of the neck portion 31.

The catheter kit 11 may further comprise a backflow prohibiting arrangement configured to prevent, or at least restrict, fluid inside the urine collection bag 3 from flowing backwards into the handle 2 or into the central lumen of the catheter shaft 1. For example, after a user has performed catheterization and the urine collection bag 3 is filled with urine the user wishes to remove the catheter shaft 1 from the urethra. During this process, the urine collection bag 3 may accidentally be compressed or held higher compared to the handle 2 or catheter shaft 1 whereby urine could flow from the urine collection bag 3 back into the handle 2 or central lumen of the catheter shaft 1 and potentially exit through the eyelets.

To prevent or at least limit this type of backflow the catheter kit 11 is provided with a backflow prohibiting arrangement. The backflow prohibiting arrangement may be a unidirectional valve which is placed in the central lumen or in the cavity of the handle 2 so as allow urine to flow in a direction from the central lumen into the urine collection bag 3 but prohibit or at least restrict flow of urine in the opposite direction. There are many types of unidirectional valves as such known in the art which may be used.

However, unidirectional valves are often costly to manufacture and may require the handle 2 to be made even larger to house the unidirectional valve. To this end, the backflow prohibiting arrangement may comprise (as an alternative to or in addition to the unidirectional valve) a flexible inner element 35 located on the inside of the urine collection bag 3 in connection with the collection bag opening 34. The flexible inner element 35 may be made of the same flexible material as the urine collection bag 3 whereby the flexible inner element 35 is arranged to be opened by a fluid flow coming from the handle 2 into the urine collection bag 3 and folded or compacted so as to prevent or at least obstruct a fluid flow coming from the urine collection bag 3 towards the handle.

The flexible inner element 35 may e.g. be a flexible inner sleeve which is circumferentially attached to the inside of the urine collection bag 3 at a proximal end and extending distally, along the axial direction A, into the urine collection bag 3 to a distal end of the flexible inner sleeve. The flexible inner sleeve may have an inner opening dimension which decreases towards the distal end wherein the flexible inner sleeve forms a funnel with a comparatively narrow distal opening 35b and comparative wide proximal opening 35a. To prohibit the flexible inner sleeve from turning inside-out during a backwards flow the flexible inner sleeve may be attached (e.g. by welding or using an adhesive) to an inside of the urine collection bag. This makes it easy for urine to flow through the flexible inner sleeve in a proximal-to-distal direction but difficult for urine to flow through the flexible inner sleeve in the opposite direction.

Other types of flexible inner elements 35 could be used as the backflow prohibiting element, examples include the backwards flow prohibiting sleeves described in European patent application number 06116966.0. Hereby incorporated by reference in its entirety. However, here the flexible inner element is advantageously formed as an integral part of the urine collection bag.

The urine collection bag 3 may further comprise one or more tear lines 39 and optionally associated tear tabs 38 allowing the urine collection bag 3 to be torn open so as to form an opening in the urine collection bag 3, allowing urine to be poured out via the formed opening. For example, a tear line 39a is provided so as to provide an opening in the main storage portion 32 of the urine collection bag 3. By providing a possibility of opening the urine collection bag 3 in the main storage portion 32 it may be easier to empty the urine collection bag 3 by e.g. holding the urine collection bag 3 by the handle 2 and pouring urine out of the urine collection bag 3.

Additionally or alternatively, a tear line 39b may be provided in the neck portion 31 allowing the urine collection bag 3 (or at least the main storage portion 32 and optionally a portion of the neck portion 31) to be torn from the handle 2. The tear line in the neck portion 31 may advantageously be arranged upstream (the direction of flow being from the catheter shaft into the urine collection bag) of the flexible inner element 35 acting to prevent back flow. In this way, when the user separates the catheter 10 from the urine collection bag 3 by tearing at the neck portion 31 the flexible inner element 35 remains in the urine collection bag so as to prevent spillage when the user e.g. ties the neck portion into a knot to dispose of the urine collection bag.

In some implementations, it is envisaged that a tear line is arranged in immediate proximity to the handle 2, leaving as much as possible of the neck portion 31 together with the urine collection after separation, making it e.g. easier to tie the neck portion 31 into a knot.

Accordingly, the urine collection bag 3 may be emptied via the neck portion 31 after it has been torn from the handle 2. Additionally or alternatively, at least a part of the neck portion 31 remains connected to the main storage portion 32 of the urine collection bag 3 after the urine collection bag 3 has been torn open at tear line 39b whereby it may be possible to tie the remaining part of the neck portion 31 into a knot, so as to seal the urine inside the urine collection bag 3 before disposing the urine collection bag 3.

Additionally, the urine collection bag 3 may further comprise one or more of external protrusions or external apertures 36 making it easier to hold the urine collection bag 3 or e.g. hang the urine collection bag 3 on a hook. The external protrusions and/or openings 36 may further make it easier for users to open the urine collection bag 3 at one or more of the tear lines by providing the user with a feature which can easily and reliantly be gripped so as to counteract the force of the tearing.

The one or more external aperture 36 may be formed in a weld region 33 surrounding the urine collection bag 3.

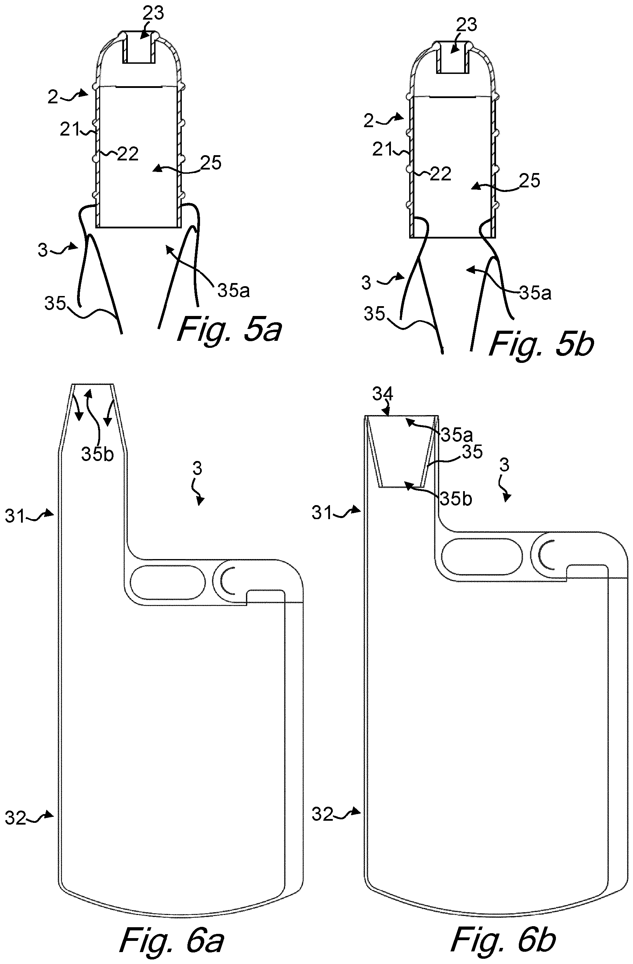

Turning to fig. 5a and 5b, two options for attachment of the urine collection bag 3 against the handle 2 are shown.

In fig. 5a the urine collection bag 3 is attached to the outer surface 21 of the handle 2. That is, the collection bag opening is attached circumferentially to the outer surface 21 of the handle 2. For example, the urine collection bag 3 is welded, attached using an adhesive or otherwise fastened to the outer surface 21 of the handle 2 so as to reliantly fixate the urine collection bag 3 to the handle 2. If the flexible inner element 35 (e.g. a sleeve) is provided to prevent or restrict backflow the flexible inner element 35 is attached to the inside of the urine collection bag with the comparatively wide proximal opening 35a placed in direct communication with the central cavity 25 or the inner cavity opening 23 through which the central lumen of the catheter shaft communicates with the cavity 25.

In the embodiment of fig. 5b the urine collection bag 3 is instead connected to the inside of the handle 2, i.e. connected to the inner surface 22 defining the cavity 25. The collection bag opening may e.g. be welded, attached using an adhesive or otherwise fastened to the inner surface 22 of the handle 2. If the flexible inner element 35 is provided to prevent or restrict backflow the flexible inner element 35 is attached to the inside of the urine collection bag with the comparatively wide proximal opening 35a placed in direct communication with the cavity 25 or the inner cavity opening 23 through which the central lumen of the catheter shaft communicates with the cavity 2.

Turning to figs. 6a and 6b, a urine collection bag 3 according to some implementations is shown. As mentioned above, the urine collection bag 3 may comprise a neck portion 31 which connects to the handle 2 at one end and connects to a main storage portion 32 at the opposite end. Additionally, the urine collection bag 3 may be manufactured so as to integrate a flexible inner element 35 acting to prevent or limit backflow of urine.

In fig. 6a the flexible inner element 35 is integrally formed with the rest of the urine collection bag 3. For example, the urine collection bag 3 may be made by folding a single sheet (e.g. a sheet of a thin plastic, such as a plastic foil) or placing two such sheets on top of each other followed by welding or adhering the (folded) sheets together along a periphery to form a urine collection bag 3 of a desired shape. The welding or adhering may be omitted at a part of the periphery, thereby forming the collection bag opening.

During manufacturing, the neck portion 31 may be realized so as to include a tubular extension wherein the tubular extension is simply a proximal extension of the neck portion 31. The tubular extension comprises an opening 35b. Optionally, the tubular extension is also tapering so as to progressively become more and more narrow along a direction away from the neck portion 31 towards the opening 35b. By folding the tubular extension into the neck portion 31 the tubular extension may be used to form a flexible inner sleeve, as shown in fig. 6b. The flexible inner sleeve is hereby attached to the urine collection bag 3 and acts as a simple backflow prohibiting element, which can be compacted and housed inside the handle together with the rest of the urine collection bag 3. Hereby, a simple and efficient method of forming a backflow prohibiting element integrated into the urine collection bag 3 is provided.

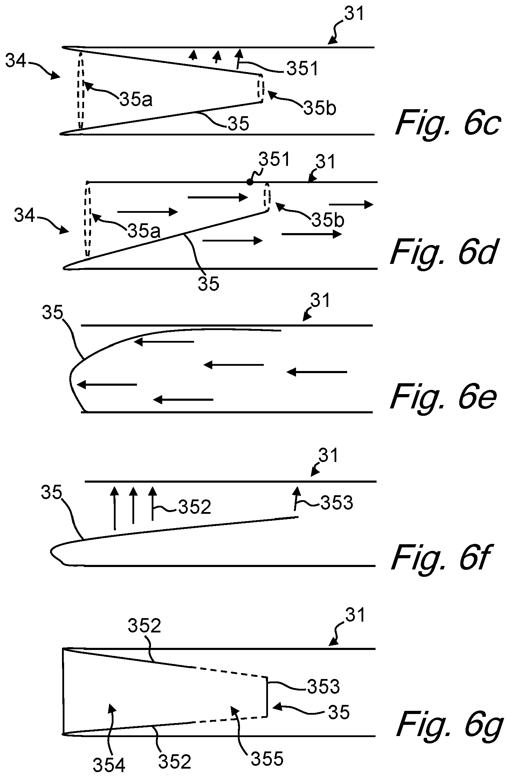

Fig. 6c shows the neck portion 31 after the tubular extension has been folded into the neck portion 31 to form a flexible inner element 35 in the form of a flexible inner sleeve. The collection bag opening 34 is formed at the same time when the tubular extension is folded inwards, into the neck portion 31. The flexible inner sleeve may further be attached to the inside of the neck portion 31 at attachment point 351 to prevent it from being turned inside out during backwards flow.

Additionally, when the tubular extension has been folded into the neck portion 31 to form the flexible inner sleeve the urine collection bag 3 may still be connected to the handle by attaching the urine collection bag 3 to the inner surface or outer surface of the handle.

The flexible inner element 35 may hereby be realized as an integral part of the urine collection bag 3.

Figs. 6d and 6e illustrate how the flexible inner sleeve may facilitate fluid flow in one direction but prevent or limit flow in the opposite direction. As seen in fig. 6d, when urine flows from the catheter shaft to the urine collection bag (to the right in fig. 6c-i) the flexible inner sleeve is opened by the flow, allowing urine to flow past the inner flexible element 35. On the other hand, when urine flows backwards, from the urine collection bag towards the catheter shaft (to the left in figs. 6c-i), the urine will force the inner opening 35b to close and trap the urine, preventing or at least restricting the backwards flow of urine, as shown schematically in fig. 6e.

The flexible inner element 35 may alternatively be made from a single layer extension, also referred to as a tongue. The tongue may e.g. be an extension of the neck portion 31 realized as an extension of the at least one sheet which has been folded, welded or glued to form the urine collection bag. The tongue is, similar to the tubular extension, folded into the neck portion 31 and connected to the inside of the neck portion 31 as shown in figs. 6f and 6g.

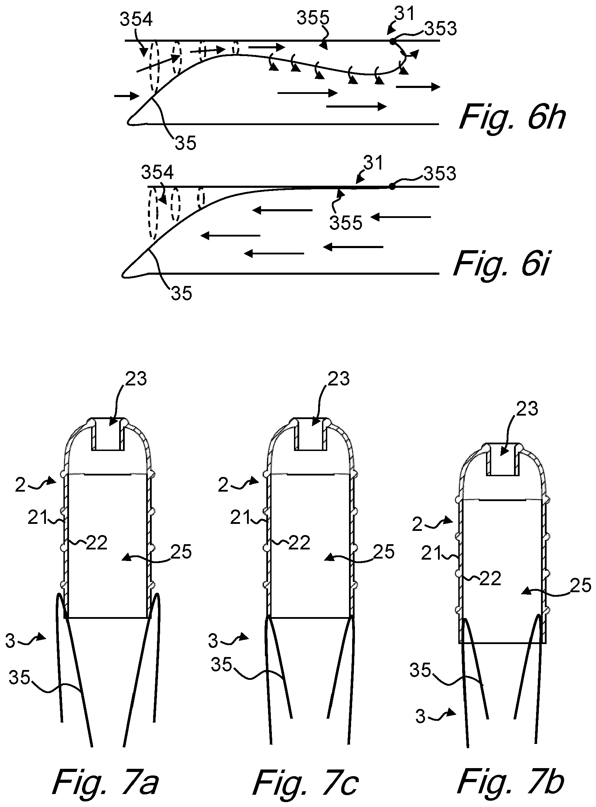

That is, the urine collection bag may be formed by folding or attaching one or two sheets together (e.g. by means of welding or by using an adhesive). One of the sheets may be made with an extension which acts like a tongue or flap that is folded inwards and arranged inside the neck portion 31 as shown in fig. 6f. The tongue is then attached to the opposite inner surface of neck portion 31. For example, the distal end of the tongue (when it is folded into the neck portion 31) is welded along a transverse welding line 353 to the opposite inner surface of the neck portion 31 and welded along substantially axial welding lines 352 to the opposite inner surface of the neck portion 31 to form a tube section 354 together with the neck portion 31. However, there is a region 355 between the transverse welding line 352 and the tube section 354 where the tongue is free from the neck portion 31. In fig. 6g the dashed lines indicate edges of the tongue which are not attached to the inside of the neck portion 31. As illustrated in figs. 6h and 6i, this structure will allow urine to flow easily in one direction but prevent or limit flow in the opposite direction. Fig. 6h illustrates how a flow from the catheter shaft into the urine collection bag (to the right in figs. 6c-i) opens region 355 by forcing the part of the tongue which is not attached to the opposite inner surface of the neck portion 31 to form an open. On the other hand, when urine flows backwards (to the left in figs. 6c-i) as shown in fig. 6i the urine will force the part of the tongue which is not attached towards the inner surface of the neck portion 31 to close region 355 and thereby prevent or prohibit urine from flowing backwards, out of the urine collection bag.

Figs. 7a-c show various examples of how the urine collection bag 3 can be attached to the handle 2, when the urine collection bag 3 is provided with a flexible inner element 35 created by folding an extension into the neck portion.

In fig. 7a the urine collection bag 3 with the flexible inner element 35 is attached to the outer surface 21 of the handle 2. The flexible inner element 35 may be attached directly to the outer surface 21 of the handle 2 and since the flexible inner element 35 is integrally formed with the urine collection bag 3 it may not be necessary to provide any further attachment between the urine collection bag 3 and the flexible inner element 35.

In fig. 7b the urine collection bag 3 is attached to the inner surface 22 of the cavity 25 in the handle 2. An outer surface of the neck portion of the urine collection bag 3 is routed into the cavity 25 and attached to the inner surface 22 of the cavity 25.

In fig. 7c the urine collection bag 3 is attached to the handle 2 at the folding point where the neck portion of the urine collection bag 3 transitions into the flexible inner element 35, wherein the flexible inner element 35 extends back into the neck portion of the urine collection bag 3.

The examples of figs. 7a-c are just a few out of many possible ways in which the urine collection bag 3 may be attached to the handle 2.

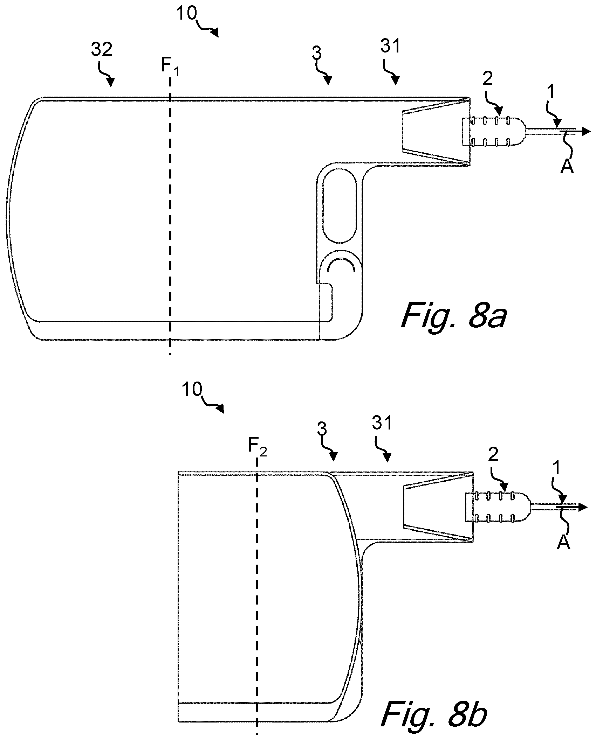

Turning to figs. 8a-e, it will now be described how the urine collection bag 3 can be compacted so as to fit inside the handle 2.

In fig. 8a a urine collection bag 3 is provided in its extended state. The urine collection bag 3 is attached to the handle 2 which in turn is connected to a catheter tube 1. The urine collection bag 3, handle 2 and catheter tube 1 together form a catheter kit 11 with an integrated urine collection bag 3 and urinary catheter 10.

The urine collection bag 3 comprises a main storage portion 32 and a neck portion 31, wherein the main storage portion 32 is communicating with the handle 2, via the neck portion 31.

To compact the urine collection bag 3 the main storage portion 31 is folded along a first transverse folding line F₁. The transverse folding line F₁ may be substantially transverse to the axial direction A and may divide the main storage portion 32 of the urine collection bag 3 approximately in half.

When the urine collection bag 3 is folded along the first transverse folding line F₁ the urine collection bag 3 comprises a folded main storage portion 32 connected to the handle 2 via the neck portion 31, as shown in fig. 8b. In some implementations, the length of the main storage portion 32 in the fully extended state along the axial direction A is between 10 and 40 cm, whereby after folding along the first transvers folding line F₁ the folded main storage portion 32 may have a length along the axial direction A of between 5 and 20 cm.

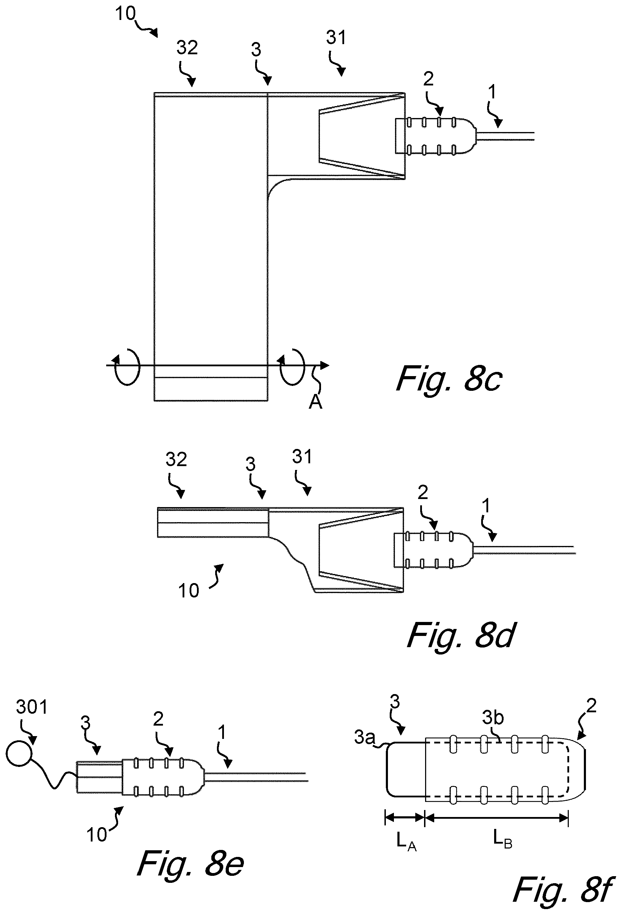

The folded main storage portion may be folded again, along a second transverse folding line F₂ which divides the folded main storage portion substantially in half. Folding along a second transverse folding line F₂ results in a double folded main storage portion 32 as shown in fig. 8c. Continuing the example of above, with a main storage portion 32 having an (unfolded) length of between 10 and 40 cm the double folded main storage portion 32 has a length of between 2.5 and 10 cm.

After two transverse folds, the double folded main storage portion 32 may exhibit a width which is greater than the length, resulting in a substantially elongated body.

For example, the main storage portion 32 may have a transverse width of at least 14 cm, or at least 16 cm and the transverse width may be substantially retained after one or more folds along transverse folding lines.

By repeatedly folding or rolling the double folded main storage portion 32 of the urine collection bag 3 along folding lines or a rolling axis which is parallel to the axial direction A it is possible to form the double folded main storage portion 32 into a compacted body, as shown in fig. 8d. Additionally or alternatively, it is envisaged that other methods of compacting the double folded main storage portion 32 along the transverse direction, perpendicular to the axial direction A could be used. For example, the double folded main storage portion 32 may be squeezed together to form the compacted body.

During rolling, repeatedly folding or otherwise compacting the double folded main storage portion 32 in the transverse direction the neck portion 31 may be partially compacted or stretched. However, since the neck portion 31 is made of a flexible material it will still allow the double folded main storage portion 32 to compact without breaking or tearing despite the main storage portion 32, after multiple folds and compactization, behaving like a comparatively stiff body.

Since the cavity of the handle 2 may have an axial length (see e.g. L₁ in fig. 2b) along the axial direction of at least 2 cm, at least 3 cm or at least 4 cm. It is now realized that the compacted body of the main storage portion 32 can be fitted into the cavity of the handle 2 as shown in fig. 8e. When inserting the compacted main storage portion into the cavity of the handle 2 the compacted main storage portion may also push in at least a part of the neck portion 31 which also gets trapped inside the cavity of the handle 2. Any elastic forces of the compacted body forcing the urine collection bag 3 to unroll or unfold will be counteracted by the compacted body being held tightly inside the cavity of the handle 2.

After inserting the compacted body of the urine collection bag 3 into the cavity of the handle 2 the compacted body may be partially, or mainly located inside the cavity of the handle 2. To make it easy for a user to pull out the compacted urine collection bag 3 from the handle 2 the compacted urine collection bag 3 may extend at least partially outside the cavity allowing the user to grip the urine collection bag 3, as shown in fig. 8e.

Fig. 8f shows a side view of the handle 2 with the compacted body of the urine collection bag 3 inserted into the cavity of the handle 2. A portion 3a of the compacted urine collection bag 3 extends on the outside of the handle 3. The length of the portion 3a of the compacted urine collection bag 3 which extends outside the handle 2 is referred to as length L_{A}. The length of the portion 3b of the compacted urine collection bag 3 which lies inside the handle 2 (see e.g. the dashed line in fig. 8f) is referred to as length L_{B}.

In some implementations, when the compacted urine collection bag 3 is inserted into the cavity of the handle 2 substantially half of the compacted urine collection bag 3 is inside the handle 2 and half is outside the handle 3, meaning that L_{A} ≈ L_{B}.

Alternatively, when the compacted urine collection bag 3 is inserted into the cavity of the handle 2 more than half of the compacted urine collection bag 3 is inside the handle 2 and less than half is outside the handle 3, meaning that L_{A} < L_{B}. To ensure that the compacted urine collection bag 3 is still easy to grip and withdraw from the handle 2 L_{A} is at least 0.5 cm or at least 1 cm.

As a further option, when the compacted urine collection bag 3 is inserted into the cavity of the handle 2 less than half of the compacted urine collection bag 3 is inside the handle 2 and more than half is outside the handle 3, meaning that L_{A} > L_{B}. While this arrangement may provide a urine collection bag 3 which is very easy to grip and withdraw from the handle 2, L_{B} may be at least 1 cm, or at least 2 cm so as to ensure that the compacted urine collection bag 3 remains inside the handle 2 and does not accidentally come loose from the handle.

Since the compacted urine collection bag 3 may substantially fill the entire length of the cavity of the handle 2 it is understood that the length L_{B} of the portion 3b of the compacted urine collection bag 3 which is inside the handle 2 may be substantially equal to the axial length L₁ (see e.g. fig. 2b) of the handle 2. For example, L₁ ≈ L_{B}.

In some implementations, the urine collection bag 3 is further provided with a gripping element 301 (see fig. 8e) configured to extend from the compacted urine collection bag when it is housed inside the cavity, to make the urine collection bag 3 easier to withdraw by pulling on the gripping element 301. The gripping element 301 may be an elongated flap, loop or thread which is attached to the urine collection bag and appropriately arranged during the folding process to be accessible from the outside of the compacted urine collection bag 3 when it is arranged in the handle.

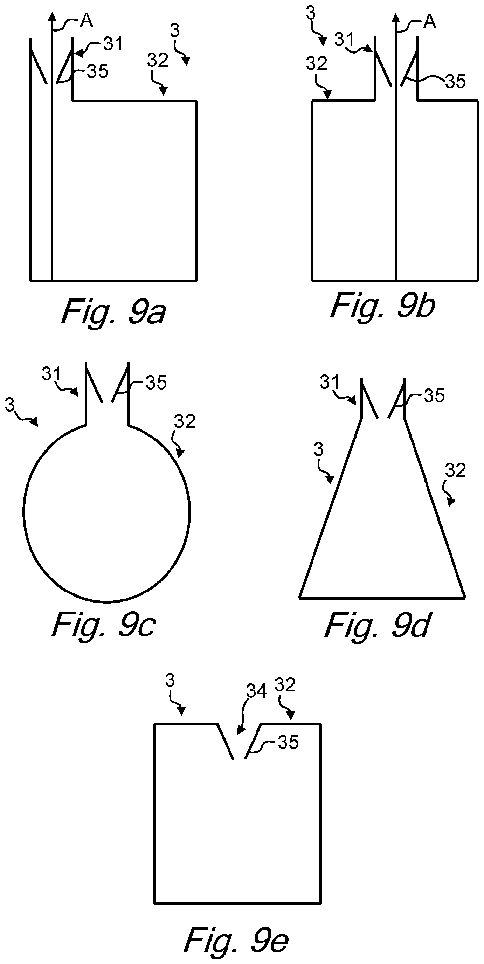

Figs. 9a-e show cross-sectional views of different urine collection bags 3 in their extended state. It will be appreciated by those skilled in the art that many other shapes may be used while still allowing the urine collection bag 3 to be compacted and stored in the handle (e.g. using the folding technique of figs. 8a-f).

In fig. 9a a urine collection bag 3 with a neck portion 31 and a main storage portion 32 connected to the neck portion 31 is shown. The urine collection bag 3 is similar to the urine collection bag 3 shown in e.g. fig. 4, figs. 6a-b and fig. 8a. This urine collection bag 3 comprises a main storage portion 32 which is transversely asymmetrical around an extension of the neck portion 31 the axial direction A which is centered in the neck portion 31. A benefit with this design of the urine collection bag 3 is that the efficient and simple folding technique of figs. 8a-f may be employed and that an asymmetrically arranged main storage portion 32 may be easier to hold during catheterization and/or while emptying the urine collection bag 3 (e.g. after opening the urine collection bag 3 via a tear line).

In fig. 9b a urine collection bag 3 with a neck portion 31 and a main storage portion 32. The main storage portion 32 of this urine collection bag 3 is symmetrical around the axial direction A running through the center of the neck portion 31. A benefit with using a symmetrical main storage portion 32 is that when urine enters into the main storage portion 32, and weights it down, the force pulling on the neck portion will be evenly distributed which e.g. may facilitate avoidance of the neck portion 31 becoming folded so as to obstruct urine flow therethrough open during catheterization.

The urine collection bags of fig. 9a and 9b have substantially rectangularly shaped main storage portions 32 but of course other shapes are possible. For example, a rounded (i.e. oval, elliptical or circular) main storage portion 32 may be used as shown in fig. 9c. Alternatively, a generally polygonal main storage portion 32 may be used. For example the main storage portion 32 may be generally triangular as shown in fig. 9d.

Irrespective of the shape of the main storage portion 32 the urine collection bag 3 may further be provided with a neck portion 31 and optionally also a flexible inner sleeve 35 acting as a backflow prohibiting element.

However, it is not strictly necessary for the urine collection bag 3 to comprise a neck portion and as seen in fig. 9e an exemplary urine collection bag 3 is presented which does not feature a neck portion. The collection bag opening 34 is provided directly into the main storage portion 32 (and optionally provided with a flexible inner sleeve 35) whereby the main storage portion 32 is attached directly to the handle 2. A urine collection bag 3 without a neck portion may still be compacted sufficiently so as to at least partially fit inside the cavity of the handle 2.

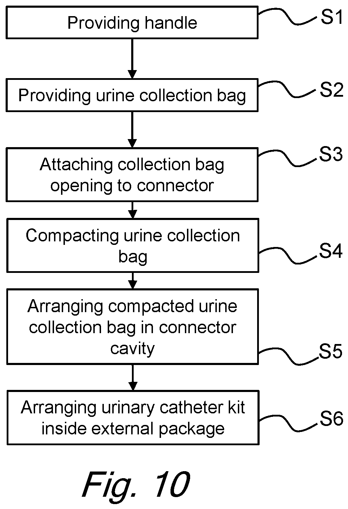

Fig. 10 is a flow chart outlining a method for manufacturing a urinary catheter kit according to some implementations. With further reference to fig. 4, the method comprises step S1 involving providing a handle 2. The handle 2 is configured to be arranged on a urinary catheter shaft 1 such that the central lumen of the catheter shaft 1 can communicate with the cavity of the handle 2.

The method then goes to step S2 comprising providing a urine collection bag 3. The urine collection bag 3 comprises a collection bag opening 34, e.g. provided in a neck portion 31 communicating with a main storage portion 32.

The method then goes to step S3 involving attaching the collection bag opening 34 to the handle 2. The attachment may be to the outer surface of the handle 2 or to the inner surface of the handle 2 forming the cavity. The attachment may be realized using a welding technique, such as laser welding, ultrasonic welding or heat welding. Alternatively, the attachment is realized by using an adhesive to fasten the urine collection bag 3 to the handle.

The method then goes to step S4 comprising compacting the urine collection bag 3 into a compacted state. For example, the compacting may comprise performing at least one of folding the urine collection bag 3 and rolling the urine collection bag 3. The urine collection bag 3 may be compacted into a shape which at least partially fits inside the cavity of the handle 2.

The method then goes to step S5 involving arranging the compacted urine collection bag 3 at least partially inside the cavity of the handle 2. Hereby a catheter kit 11 comprising a catheter 10, with a handle 2 housing a urine collection bag 3 has been formed. The catheter kit 11 is a single piece as seen in e.g. fig. 1. The catheter kit 11 may be provided to a user which can easily withdraw the urine collection bag 3 from the handle 2, extend the urine collection bag 3 and perform catheterization with the urine being collected in the urine collection bag 3.

To form a complete catheter kit 11, the handle 2 must be connected to a catheter shaft and it is envisaged that attaching the handle 2 to a catheter shaft 1 may occur at any time in relation to steps S1-S5. For example, the handle 2 may already be arranged on the catheter shaft 1 at step S1 where step S1 involves provision of a catheter comprising the handle 2 and the catheter shaft 1 already attached to the handle 2. As another example, the handle 2 is attached to the catheter shaft after step S5 meaning that the catheter shaft 1 is attached to the handle 2 when the urine collection bag 3 is already housed inside the cavity of the handle 2. This enables e.g. the handle 2 and urine collection bag 3 to be manufactured separately and connected to the catheter shaft 1 later. As yet another example, the catheter shaft 1 is attached to the handle 2 between steps S3 and S4 meaning that when the catheter shaft 1 is attached to the handle 2, the urine collection bag 3 has already been attached to the handle 2, but not yet compacted and housed in the cavity.

Additionally, it is envisaged that some processing of the catheter shaft 1 may be performed before or after the catheter shaft 1 has been attached to the handle 2. The processing may involve one or more of coating the catheter shaft (e.g. with a hydrophilic coating), forming a rounded tip at the insertion end and forming the one or more eyelets 14.

Provided the catheter shaft has been attached to the handle 2 (and optionally been subject to one or more of the catheter shaft processing steps as described above) at some time in relation to steps S1-S5 to form the complete catheter kit 11, the method may further comprise step S6 involving placing the catheter kit 11 inside an external package which serves to protect the catheter kit 11 and keep it sterile. As described above, the catheter shaft 1 may be coated with a hydrophilic coating configured to be wetted before it is used. Hereby, the external package may store a catheter kit 11 which is already wetted, and therefore ready to use immediately, or it is envisaged that a sachet with a wetting liquid is placed in the external package whereby the user may use the sachet to wet the hydrophilic coating layer.

The person skilled in the art realizes that the present invention by no means is limited to the preferred embodiments described above. On the contrary, many modifications and variations are possible within the scope of the appended claims. For example, while urine collection bags with one flexible inner element acting as a backflow preventing arrangement has been described, it is envisaged that multiple flexible inner elements may be used (e.g. arranged in series in the neck portion) to provide even better backflow prevention. Since the flexible inner elements may be realized using a thin material (e.g. the same material as that of the urine collection bag) multiple flexible inner elements can be used without prohibiting the compacted urine collection bag from being inserted into the cavity of the handle. Additionally or alternatively one more flexible inner elements may be used together with one or more unidirectional valves provided in the handle, to facilitate better backflow prevention.

In the claims, the word "comprising" does not exclude the presence of other elements or steps than those listed in the claim. The word "a" or "an" preceding an element does not exclude the presence of a plurality of such elements.

## Claims

1. A urinary catheter kit comprising:
a urinary catheter comprising:
- a catheter shaft extending along an axial direction, from a proximal insertion end to a distal end, wherein said catheter shaft comprises a central lumen,
- at least one eyelet arranged at the insertion end, wherein said eyelet communicates with the central lumen,
- a handle arranged at the distal end of the catheter shaft, wherein said handle defines a cavity having an inner cavity opening which is in fluid communication with the central lumen, and
a urine collection bag made of a flexible material,
wherein the urine collection bag has a collection bag opening which is attached to the handle so as to enable the urine collection bag to communicate with the central lumen via the inner cavity opening,
wherein the urine collection bag, in a storage state, is compacted and at least partially housed inside the cavity of the handle.

2. The catheter kit according to claim 1, wherein the urine collection bag opening is attached to an inner surface of the cavity.

3. The catheter kit according to claim 1, wherein the urine collection bag opening is attached to an outer surface of the handle.

4. The catheter kit according to any of the preceding claims, wherein the handle and the cavity extends in the axial direction and wherein a cross-section of the cavity in a plane perpendicular to the axial direction is oval.

5. The catheter kit according to any of the preceding claims, further comprising:
a backflow prohibiting arrangement, configured to prevent or limit a liquid flow from the urine collection bag into the central lumen of the catheter shaft while allowing a liquid flow from the central lumen of the catheter shaft into the urine collection bag.

6. The catheter kit according to claim 5, wherein the backflow prohibiting arrangement comprises a flexible inner element attached at a proximal end to the urine collection bag and extending distally into the urine collection bag.

7. The catheter kit according to claim 6, wherein the urine collection bag comprises a neck section and wherein the flexible inner element is arranged at least partially inside said neck section.

8. The catheter kit according to claim 6 or claim 7, wherein the flexible inner element is integrally formed with the urine collection bag.

9. The catheter kit according to any of claims 5 - 8, wherein the backflow prohibiting arrangement comprises a unidirectional valve arranged in the handle.

10. The catheter kit according to any of the preceding claims, wherein the handle extends in the axial direction, and wherein a largest axial length of the cavity is at least 2.5 cm, at least 3 cm or at least 3.5 cm and/or wherein a largest cross-sectional dimension of the cavity in a plane perpendicular to the axial direction is at least 1 cm, or at least 1.5 cm.

11. The catheter kit according to any of the preceding claims, wherein the urine collection bag comprises a neck portion and a main storage portion, wherein a transverse width of the urine collection bag in the main storage portion is greater than a transverse width of the neck portion and wherein the neck portion is arranged proximally of the main storage portion, between the collection bag opening and the main storage portion.

12. The catheter kit according to any of the preceding claims, wherein the main storage portion, in the storage state has been folded at least once along a transverse folding line, the transverse folding line being perpendicular to the axial direction when the urine collection bag is in the extended state, and subsequently rolled, or repeatedly folded, along a roll axis parallel to the axial direction.

13. The catheter kit according to any of the preceding claims, wherein the urine collection bag further comprises at least one tear line configured to enable the urine collection bag to be torn open.

14. A urinary catheter assembly, comprising:
an external package, and
a catheter kit according to any of the preceding claims arranged inside said external package.

15. A method for manufacturing a handle for a catheter kit comprising:
providing a handle configured to be arranged at the distal end of a catheter shaft, wherein said handle defines a cavity having an inner cavity opening which is configured to be in fluid communication with a central lumen of the catheter shaft;
providing a urine collection bag comprising a collection bag opening;
attaching the collection bag opening to the handle of the urinary catheter;
compacting the urine collection bag; and
arranging the compacted urine collection bag inside the cavity of the handle.
